# EUROPÄISCHE PATENTSCHRIFT

(11) **EP 2 716 320 B1**
(45) Veröffentlichungstag und Bekanntmachung des Hinweises auf die Patenterteilung: **25.12.2019**
(21) Anmeldenummer: 13005484.4
(22) Anmeldetag: 18.05.2010
(51) Int. Cl.: A61M 16/06

(54) **Vorrichtung zur Positionierung eines Patienten Interfaces**
Device for positioning a patient interface
Dispositif de positionnement d'une interface de patient

(30) Priorität: 18.05.2009 DE 102009021807
(43) Veröffentlichungstag der Anmeldung: 09.04.2014
(62) Teilanmeldung aus: 10727645.3
(73) Patentinhaber: Löwenstein Medical Technology S.A., 2557 Luxembourg (LU)
(72) Erfinder: Eifler, Martin, 25348 Glückstadt (DE); Hahn, Henry, 22525 Hamburg (DE); Gardein, Joachim, E-38430 Icod de los Vinos Tenerife/Islas Canarias (ES); Wonslyd, Anne, 20259 Hamburg (DE); Feldhahn, Karl-Andreas, Dr., 22761 Hamburg (DE)
(74) Vertreter: Marx, Thomas

(56) Entgegenhaltungen:
- EP-A2- 1 493 461
- WO-A2-2004/021960
- WO-A2-2008/036625
- AU-A1- 2007 221 773
- DE-C1- 10 057 893
- US-A1- 2007 240 721

## Beschreibung

Die Erfindung betrifft eine Vorrichtung zur Beatmung mit einer Befestigungseinrichtung zur Befestigung eines Patienten Interfaces am Kopf des Anwenders.

Patienten Interfaces dienen dazu, von einem Beatmungsgerät, bereitgestelltes Atemgas an den Patienten abzugeben. Patienteninterfaces können in unterschiedlichen Ausführungsformen beispielsweise als Sauerstoffbrillen, Nasal Pillow-, Nasal- oder Vollgesichtsmasken realisiert sein. Das Patienten Interface ist typischerweise über einen Atemgasschlauch mit dem Beatmungsgerät verbunden und wird am Kopf des Anwenders fixiert.

Eine genaue Passform des Patienten Interfaces sowie der Befestigung ist erforderlich um eine Therapie-Beeinträchtigung durch z.B. Verrutschen oder Lösen zu vermeiden.
Zur Gewährleistung einer sicheren Positionierung der Atemmaske im Bereich des Gesichtes eines Patienten, sowie der Verringerung der Kräfte die auf das Gesicht wirken, werden Atemmasken mit Stirnabstützungen benutzt. Derartige Stirnstützen besitzen typischerweise Höhen- oder Abstandsverstellmöglichkeiten. Viele Verstelleinrichtungen sind für den Patienten aber oft zu kompliziert und sind meist nur aufwendig vom Patienten oder im Schlaflabor einstellbar. Eine direkte Anpassung beim Tragen der Maske ist meist nicht möglich.

Durch die zusätzliche Verwendung einer Stirnstütze an dem gaszuführenden Patienten Interface, wie z.B. einer Nasalmaske, wird die sichere Positionierung der Atemmaske im Bereich des Gesichtes eines Patienten gewährleistet. Ein ungewolltes Ablösen oder Verrutschen der Maske vom Gesicht des Benutzers und daraus folgende Leckagen werden vermieden. Das Verrutschen eines schlecht sitzenden Patienten Interfaces kann zu unangenehmen Druckstellen bis hin zu Therapiebeeinträchtigungen bzw. Therapieunterbrechungen führen. Sowohl der Tragekomfort als auch die Stabilität werden durch das Verwenden einer Stirnabstützung erhöht. Es lassen sich Druckstellen im Anlagebereich der Maske vermeiden, die bei einer Beatmungsmaske ohne Stirnabstützung durch die Vorspannung der Bänderung entstehen würden. Um den Druck auf den Nasenrücken zu verringern, ist jedoch eine individuelle Einstellung der Stirnstütze zur Maske erforderlich.
Die WO 2008/036625 A2 und auch die EP 2005987 A2 offenbaren jeweils eine Beatmungsmaske mit einer zweiteiligen Stirnstützenverstellung mit einem Stirnstützenträgerarm und einem Stirnstützenschaft im Maskenkörper. Der bewegliche Stirnstützenträgerarm ist hier schwenkbar um eine Achse im Maskenkörper gelagert. Im Bereich einer Aussparung des Stirnstützenträgerarmes sind mehrere Rastpositionen vorhanden, die durch korrespondierende Rastmittel des Stirnstützenschafts belegt werden können. Der Abstand der Stirnstütze wird vom Nutzer manuell durch Verschwenken und Einrasten des Stirnstützenträgerarmes festgelegt.
Die AU 2007221773 A1 und auch WO 2004/021960 A2 offenbaren jeweils eine Beatmungsmaske mit einer Stirnstützenverstellung, bei der ein beweglicher Stirnstützenträgerarm an einer Fläche verschiebbar im Stirnstützenschaft des Maskenkörpers gelagert ist. Der Abstand der Stirnstütze wird vom Nutzer manuell festgelegt, indem der Stirnstützenträgerarm relativ zu dem Stirnstützenschaft verschoben und in der gewünschten Position fixiert wird.
Aufgabe der vorliegenden Erfindung ist es daher, eine Vorrichtung zur Beatmung der einleitenden Art derart zu konstruieren, dass eine funktionelle, einfach zu bedienende Verstellung der Stirnabstützung bereitgestellt und dabei eine hohe Funktionalität und Robustheit gegeben wird.

Diese Aufgabe wird erfindungsgemäß gelöst, durch eine Vorrichtung zur Beatmung, zumindest bestehend aus einem Körper, welcher einen Innenraum von einem Außenbereich abgrenzt, einem Bereich zur Abdichtung der Vorrichtung gegen Gesichtspartien eines Patienten, Befestigungselementen zur Positionierung der Vorrichtung am Kopf des Patienten und einem Anschluss zur Zufuhr von Atemgas, wobei die Vorrichtung zur Beatmung über ein Stellglied verfügt, das beim Verstellen einen Abstand mindestens eines Teiles der Vorrichtung zu mindestens einem Körperbereich des Patienten verändert, wobei die Vorrichtung eine Stirnstütze, einen Stirnstützenträger und einen Stirnstützenschaft aufweist, wobei der Stirnstützenschaft am Maskenkörper integriert ist, wobei der Stirnstützenträger einen Arm aufweist der geschlitzt oder geteilt ist und ein federndes Element mit einem Verstellknopf aufweist, wobei der Arm seitliche, gebogene Abrollflächen aufweist, die entweder glatt oder gezahnt sind, wobei das Stellglied das federnde Element mit dem Verstellknopf und den seitlichen Abrollflächen umfasst, wobei eine Verstellmöglichkeit der Stirnstütze über das Abrollen der seitliche, gebogenen Abrollflächen am geschlitzten oder geteilten Arm des Stirnstützenträgers auf jeweils einer entsprechenden glatten oder gezahnten Fläche außen am Stirnstützenschaft ermöglicht ist und die Fixierung oder Blockierung der Abrollbewegung über eine Geometrie erfolgt, bei der das federnde Element Rastnasen aufweist, die die Abrollbewegung blockieren, indem sie in verschiedene Nuten am Stirnstützenschaft einrasten und so die Stirnstütze in der gewünschten Position fixieren.

Die Veränderung des Abstandes kann in unterschiedlichen Varianten erfolgen. Gemeinsam ist allen nachfolgend beschriebenen Ausführungsformen, daß eine für den Patienten einfache Bedienung erfolgen kann, eine hohe Robustheit der Vorrichtung erreicht ist und trotzdem eine unbeabsichtigte Verstellung vermieden wird.

In den Zeichnungen sind Ausführungsbeispiele schematisch dargestellt. Es zeigen:
Fig. 1 bis 3: Perspektivansichten von Masken mit Detailschnitten durch die Verstelleinheit,
Fig.4: eine Perspektivansicht einer Maske mit Detailansichten des Verstellbereiches,
Fig. 5 bis 8: Schematische Darstellungen von Masken mit Verstellmöglichkeiten über schiefe Ebenen,
Fig. 9: Perspektivansichten einer Maske in zwei Stellbereichen mit Längsschnitten,
Fig.9.1: eine Perspektiv- und Seitenansicht einer Maske,
Fig. 10, 11: Schematische Darstellungen einer Maske mit Verstellmöglichkeiten senkrecht oder unter einem bestimmten Winkel alpha,
Fig.12: Seitenansichten einer Maske in zwei Vertellbereichen und Explosionsdarstellungen der Einzelteile der Stirnstützenverstellung,
Fig. 12.1: eine perspektive Darstellung einer Stirnstützenverstellung und Explosionsdarstellungen der Bajonettverbindung,
Fig. 13: Ansichten eines Stirnstützenträgers mit Verstelleinrichtung,
Fig. 14: eine perspektivische Darstellung einer Maske mit Oberkopfauflage und Detail der Verstellung,
Fig. 15: eine Perspektiv- und Explosionsdarstellung einer Klemmverbindung und
Fig. 16: eine perspektivische Seiten- und Innenansicht einer Klemmvorrichtung.

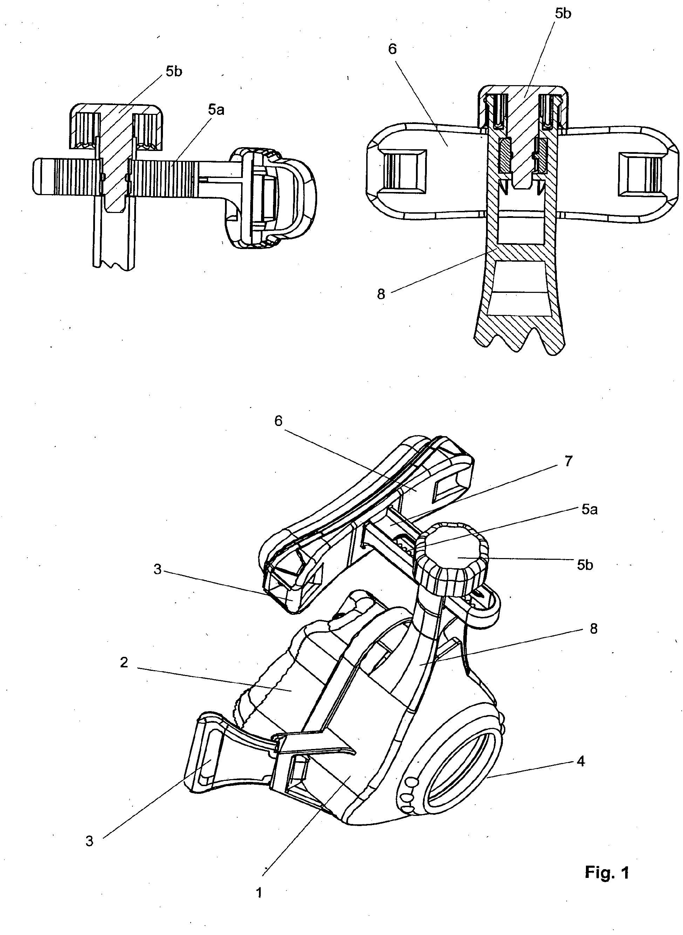

In einer Ausführungsform der Stirnstützenverstellung (Fig. 1) erfolgt die Justierung der Stirnstütze (6) über eine Zahnstange (5a). Die Zahnstange (5a) ist im Arm (7) des Stirnstützenträgers (6) angeordnet und ist so ausgeführt, dass sie außen eine glatte Oberfläche aufweist und innen eine längliche Öffnung, ähnlich einer Nut, mit kleinen Zähnen an einer Seite. Im Schaft der Stirnstütze (8), welcher am Maskenkörper (1) integriert ist, befindet sich eine Öffnung zur Aufnahme des Arms des Stirnstützenträgers (7). In diese wird der Arm des Stirnstützenträgers (7) eingeführt und kann sich dort vor- und zurück bewegen. Von außen wird durch eine weitere Öffnung im Stirnstützenschaft (8) die Verstelleinheit (5b), die auf der Achse ein kleines Zahnrad besitzt, durch die Zahnstange (5a) durchgeführt und im Schaft der Stirnstütze (8) verrastet. Durch Drehen der Verstelleinheit (5b) bewegt sich das Zahnrad in der Zahnstange (5a) und verstellt so die Stirnstütze (6) relativ zum Maskenkörper (1) vor und zurück. Durch eine leichte Demontage der Verstelleinheit kann man den Arm des Stirnstützenträgers (7) entnehmen. Dieser Arm ist in der Höhe asymmetrisch am Stirnstützenträger (6) angeordnet und bietet durch ein Drehen des Stirnstützenträgers (6) um 180° eine einfache Höhenverstellung der Stirnstütze. Das Handling ist einfach und besteht nur aus wenigen einfachen Teilen.
Die Fixierung der Stirnabstützung am Patientenkopf erfolgt über handelsübliche Kopfbänderungen oder -hauben, die an den Seiten des Stirnstützenträgers (3) befestigt werden.

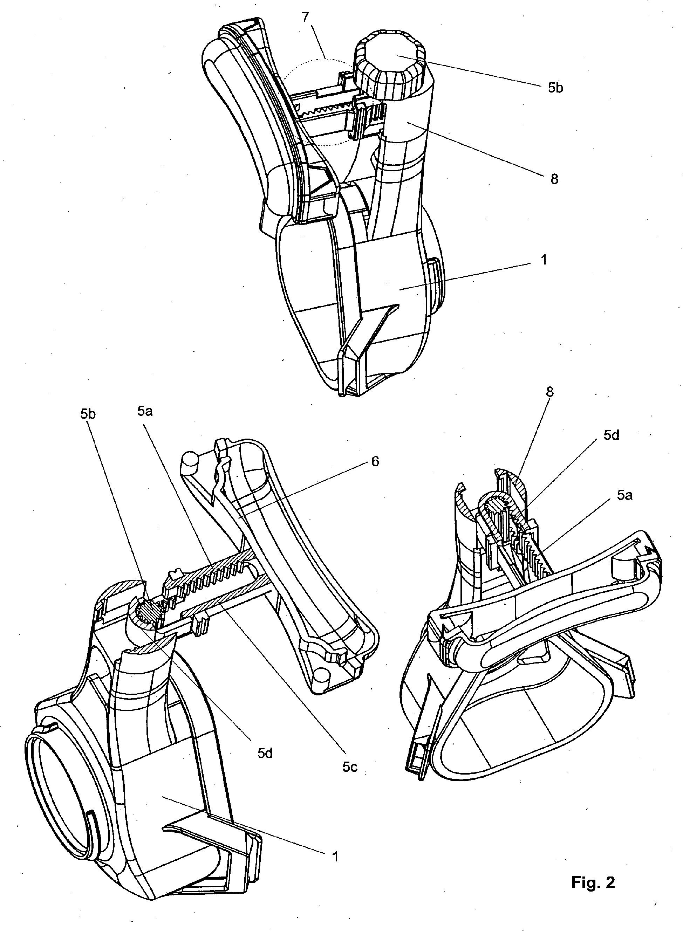

In einer etwas abgewandelten Form der Zahnstangenverstellung (Fig.2) wird am Stirnstützenträger (6) anstelle des einteiligen Armes, eine offene Zahnschiene (5a) und zur Führung eine weitere glatte Schiene (5c) angebracht. Sie sind am Stirnstützenträger (6) in der Höhe asymmetrisch außerhalb der Mitte angeordnet. Die Zahnschiene bildet das erste Verstellteil der Zahnstangenverstellung. Ein U-förmiges zweites Zahnteil (5d) bildet zusammen mit der Zahnschiene (5a) und der Schiene (5c) den Arm des Stirnstützenträgers, der teleskopartig in-, bzw. auseinander gefahren werden kann. Im zweiten Zahnteil (5d) befindet sich eine Aufnahme für die Führungsschiene (5c) und eine Öffnung, in die die erste Zahnschiene (5a) eingeführt wird. Die Außenseite der ersten Zahnschiene (5a) weist eine Rastnase auf, die in die Öffnung des zweiten Zahnteiles (5d) fast. Beides zusammen wird in die Öffnung des Stirnstützenschaftes (8) eingesetzt und von außen wird durch eine weitere Öffnung im Stirnstützenschaft (8) die Verstelleinheit (5b), die auf der Achse eine Verzahnung aufweist, durch die Zahnstange (5d) durchgeführt und im Schaft der Stirnstütze (8) verrastet. Durch Drehen der Verstelleinheit (5b) bewegt sich die verzahnte Achse der Verstelleinheit (5b) in der zweiteiligen Zahnstange und verstellt so die Stirnstütze (6) relativ zum Maskenkörper (1) vor und zurück. Dieses funktioniert teleskopartig in zwei Stufen. Zuerst bewegt sich beim Drehen der Verstelleinheit (5b) nur das erste Zahnschienenteil (5a) und erst, wenn die Rastnase des ersten Zahnteiles (5a) am Anschlag der Öffnung des zweiten Zahnteiles (5d) anliegt, nimmt er das zweite Zahnteil (5d) mit. Das erste Zahnteil (5a) fügt sich teleskopartig in das zweite Zahnteil (5d) ein. Durch das teleskopartige Ineinander fahren der zwei Zahnteile weist der Arm des Stirnstützenträgers (7) eine sehr kleine Bauweise auf.

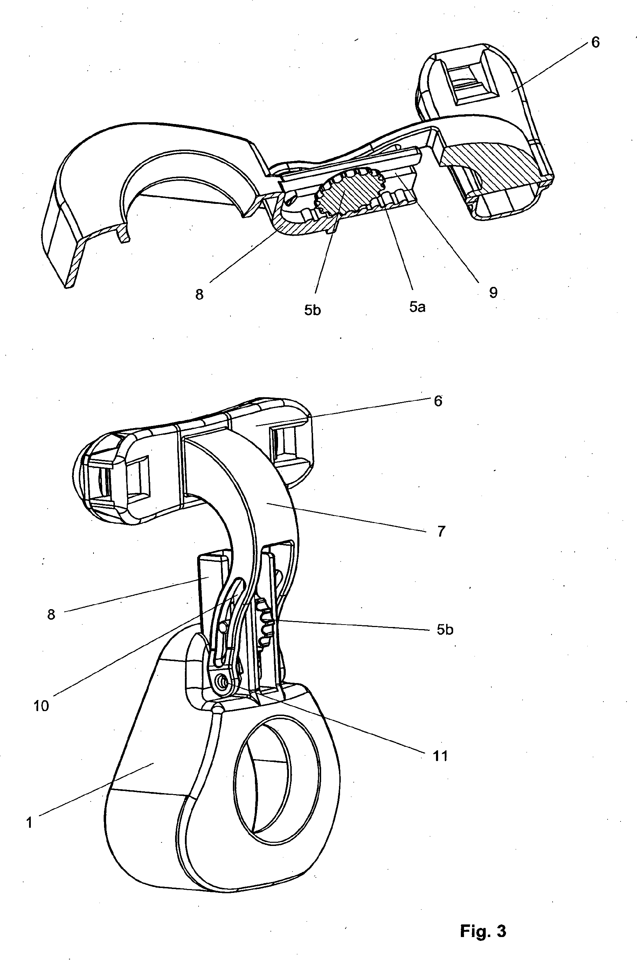

Eine weitere Ausführungsform der Zahnstangenverstellung (Fig. 3) wird durch eine verzahnte Lauffläche (5a) im Innenbereich des Stirnstützenschaftes (8) realisiert. Der Stirnstützenschaft (8), der am Maskenkörper (1) integriert ist, ist hohl, vorne geöffnet, weist innen eine Zahnlauffläche (5a) auf und ist an den Seiten geschlitzt. Die Schlitze (9) dienen der Führung eines Zahnrades (5b). Das Zahnrad (5b) dient der Verstellung der Stirnstütze (6). Es ist so ausgeführt, dass es an beiden Seiten Achsen aufweist, welche in den Schlitzen (9) des Stirnstützenschaftes (8) und gleichzeitig in den gebogenen Nuten (10) seitlich im Arm des Stirnstützenträgers (7) laufen. Der Arm des Stirnstützenträgers (7) ist in der Seitenansicht S-förmig gebogen, im unteren Bereich geschlitzt und weist an den Enden zu beiden Seiten Verrastungen auf, die in entsprechende Öffnungen im Stirnstützenschaft (8) fassen. Durch das Drehen des Zahnrades (5b) verstellt sich die Stirnstütze (6) relativ zum Maskenkörper (1) um den Verrastpunkt (11) im Stirnstützenschaft (8) vor und zurück. Die virtuelle Achse des Zahnrades führt eine gestreckte Bewegung aus und bewirkt durch die Form des Stirnstützenträgers (7) eine Verstellung in Richtung der Stirn des Patienten.

Unter einer gestreckten Bewegung sind Bewegungen zu verstehen, die linear oder in einem großen Bogen erfolgen. Dabei kann der Bogen einer Kreisbahn oder einer beliebigen Bahn entsprechen. Eine gestreckte Bewegung liegt insbesondere dann vor, wenn der Radius der Bewegung zumindest eines Punktes des Stellgliedes zumindest auf einem Teilsegment der Bewegungsbahn größer als etwa 25 mm ist. In einer bevorzugten Ausführungsform ist der Radius der Bewegung zumindest eines Punktes des Stellgliedes zumindest auf einem Teilsegment der Bewegungsbahn größer als etwa 50 mm. Besonders bevorzugt ist ein Radius der Bewegung zumindest eines Punktes des Stellgliedes zumindest auf einem Teilsegment der Bewegungsbahn größer als etwa 75 mm.

Eine gestreckte Bewegung liegt auch dann vor, wenn die Bewegung eines Stellgliedes eine Überlagerung mehrerer Bewegungsformen ist. So entspricht das Abwälzen eines Zahnrades auf einer linearen oder gebogenen Kontur einer Rotation um die (ggf. virtuelle und ortsbewegliche) Zahnradachse bei gleichzeitigem Verschieben des Zahnrades entlang der Bahn der Wälzpartner. In diesem Fall kann die (ggf. virtuelle und ortsbewegliche) Achse des Zahnrades eine gestreckte Bewegung im Sinne der oben stehenden Definition ausführen. Eine (ggf. virtuelle und ortsbewegliche) Achse ist ein Teil des Stellgliedes und kann somit eine gestreckte Bewegung im Sinne der oben stehenden Definition ausführen.

Weiterhin liegt eine gestreckte Bewegung vor, wenn ein Punkt des Stellgliedes eine gestreckte Bewegung im Sinne der oben stehenden Definition ausführt, während das Stellglied selbst eine beliebige komplexe Bewegung ausführen kann.

In bevorzugten Ausführungsformen erfolgt die Bewegung des Verstellelementes nicht proportional zur Bewegung, die die Stirnauflage vollzieht. Dieses erfolgt zumindest in Teilbereichen des Stellweges und lässt sich auf alle in dieser Schrift angeführten Ausführungsformen anwenden.
Durch die nicht proportionale Übertragung der Bewegung wird z.B. im mittleren Stellbereich eine feinere Einstellung als in dem äußeren Stellbereichen ermöglicht.

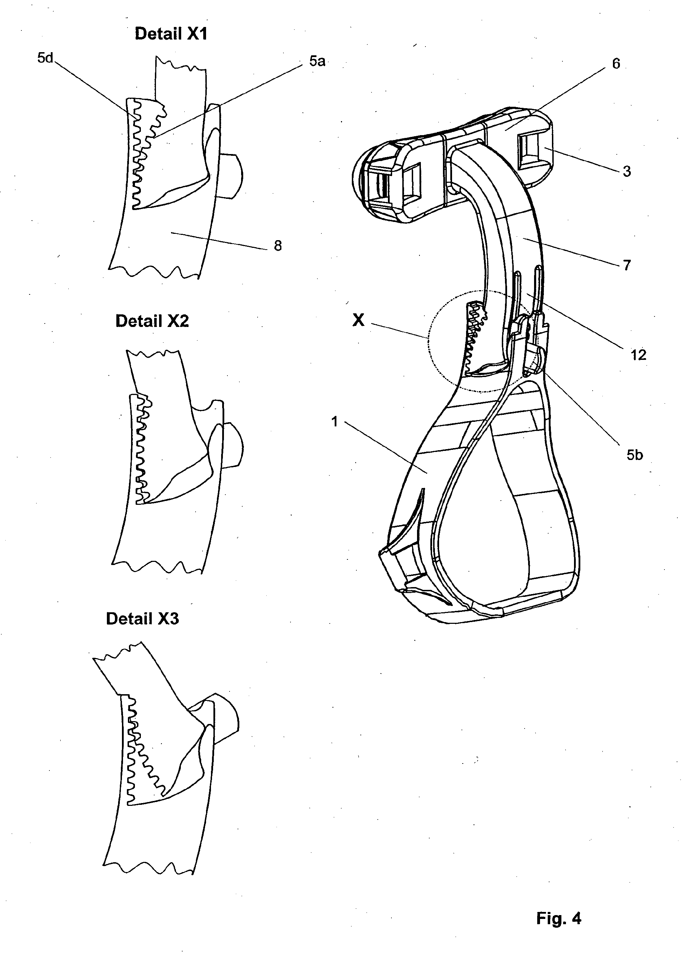

Fig.4: In einer weiteren Ausführungsvariante mit einer verzahnten Verstellmöglichkeit der Stirnstütze (6) wird dieses über das Abrollen gebogenen Verzahnungen (5a) am geschlitzten Arm des Stirnstützenträgers (7) auf jeweils einer Zahnfläche (5d) außen am Stirnstützenschaft (8) ermöglicht. Möglich wäre auch das Abrollen auf einer glatten Fläche. Das Abrollen über eine verzahnte Fläche dient der besseren Führung. Der Arm des Stirnstützenträgers (7) ist geteilt, weist ein federndes Element (12) mit einem Verstellknopf (5b) und seitliche Abrollflächen (5a) auf. Die Fixierung oder Blockierung der Abrollbewegung erfolgt über eine zweite Geometrie (dargestellt in den Detailansichten X1 bis X3). Das federnde Element (12) weist Rastnasen auf, die die Abrollbewegung blockieren, indem sie in verschiedene Nuten am Stirnstützenschaft (8) einrasten und so die Stirnstütze (6) in der gewünschten Position fixieren. Es handelt sich hierbei um keine lineare Vor- und Rückverstellung der Stirnstütze (6), sondern über eine gleichzeitige Verstellung der Höhe und des Abstandes der Stirnstütze (6) zum Kopf des Patienten, die nicht kreisförmig sondern annähernd einer Evolvente ausgebildet ist. Dieses resultiert daraus, dass keine feste Rotationsachse vorgegeben ist.

Als Alternative zur Einstellung der Stirnstütze über verzahnte Verstellmöglichkeiten bietet es sich an, den Stirnstützenschaft in mehrere Elemente zu unterteilen und diese über das Verschieben mindestens eines Elementes auf einer geneigten, schiefen Ebene zu verstellen. Die Verstellung ist variabel auf der schiefen Ebene positionierbar und so für den Benutzer auf seine individuelle Gesichtstopographie einstellbar. Der besondere Vorteil für den Benutzer ist die gleichzeitige Höhen- als auch Tiefenänderungen der Stirnstütze. So können sämtliche anatomischen Merkmale, wie z.B. Überaugenwülste und auch ethnisch bedingte topografische Unterschiede ausgeglichen werden. Ausführungsbeispiele werden in den folgenden Figuren beschrieben.

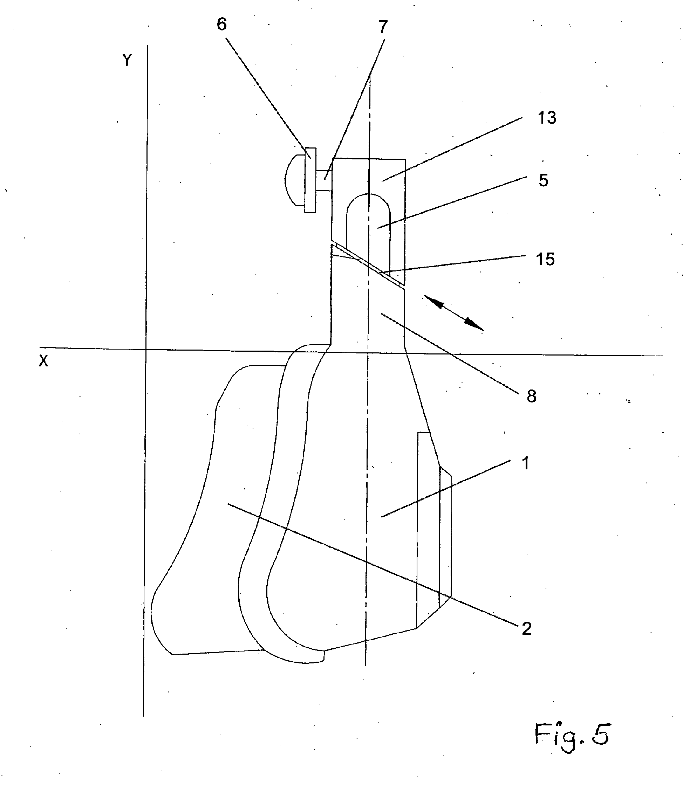

Die Stirnstütze aus Fig.5 besteht aus min. zwei Verstellelementen, die gegeneinander verschiebbar sind. Der Stirnstützenschaft (8) ist mit dem Gaszuführenden PI im Bereich des Maskenkörpers (1) verbunden und bildet das erste Verstellelement. Das zweite Verstellelement (13) ist mit der Stirnstütze (6) über den Arm (7) verbunden. Mindestens eins der Elemente verfügt über Möglichkeiten der Fixierung am Kopf z.B. Ösen oder Clips zur Aufnahme einer Bänderung oder einer Haube.

Die jeweils aufeinander liegenden Flächen der zwei Verstellelemente stehen parallel zueinander und weisen einen Winkel zwischen 1° und 89° zur X-Achse auf, so dass jeweils eine schiefe Ebene (15) entsteht. Entlang dieser Ebene (15) sind die Elemente (8, 13) zueinander in min zwei variablen Positionen verschieb- und arretierbar. Bei einem Verschieben des zweiten Elementes (13) entlang der Gleitfläche (15) relativ zum Stirnstützenschaft (8), verändert sich der Abstand der Stirnstütze (6) zur X-Achse derart, dass die Stirnstütze (6) parallel zur Y-Achse vor- und zurück bewegt wird. Gleichzeitig wird das zweite Verstellelement (13) in der Höhe bezogen auf die Y-Achse verstellt. Solange vom Benutzer leichter Druck auf die gewölbten Flächen (5) des Verstellelementes ausgeübt wird, können der Stirnstützenschaft (8) und das Verstellelement (13) gegeneinander entlang der Gleitfläche (15) verschoben werden. An mindestens einem der Endpunkte der Gleitfläche des Verstellteiles (13) und/oder des Stirnstützenschaftes (8) ist ein Anschlag zum Sperren angeordnet, sodass die Teile nicht völlig auseinander rutschen können.

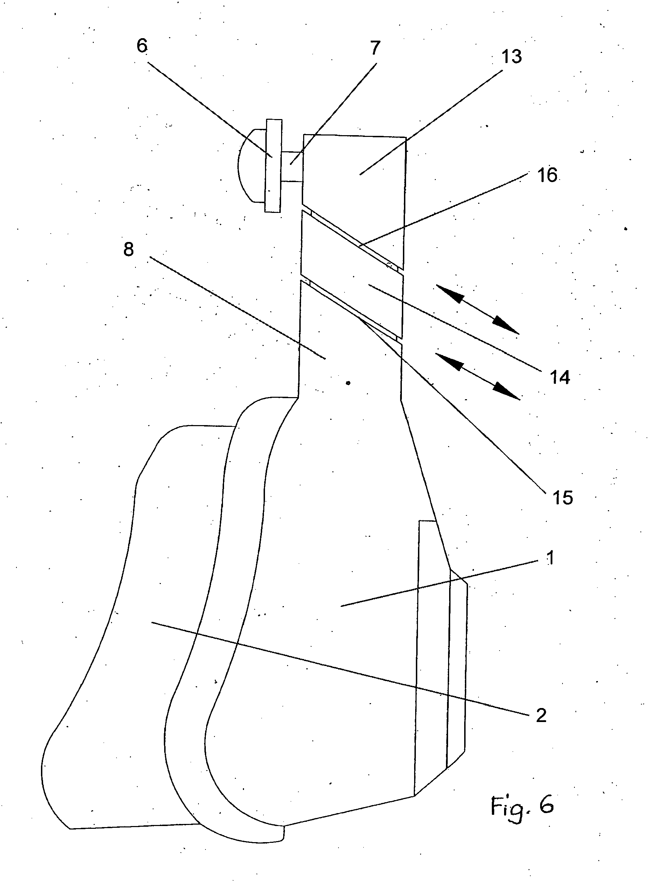

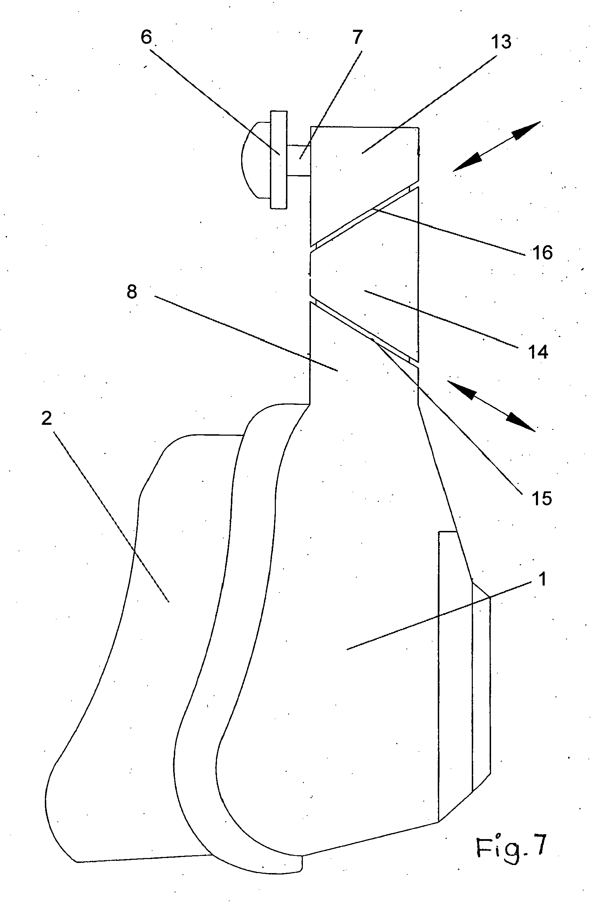

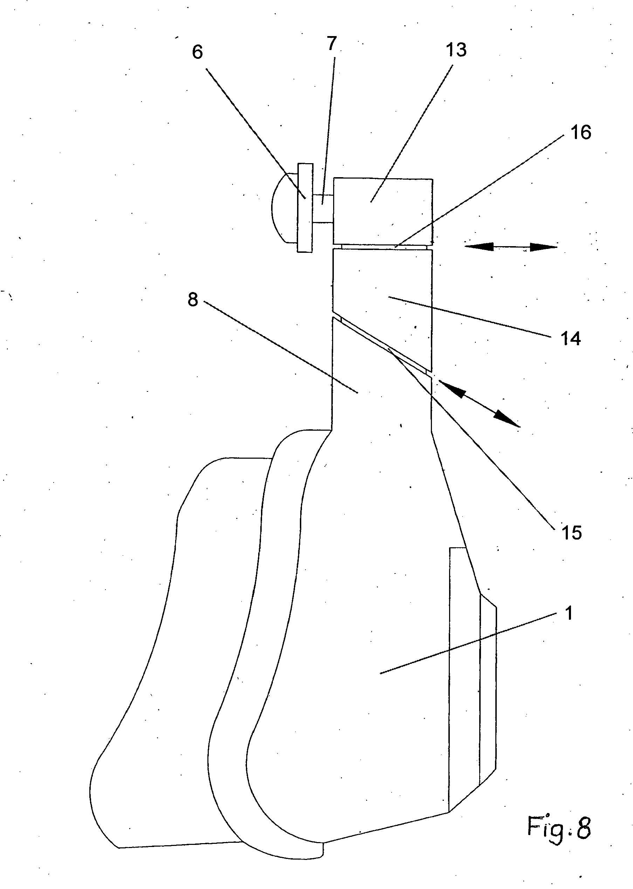

Die Beatmungsmasken mit Stirnabstützung gemäß der Fig.6, Fig.7 und Fig.8 bestehen aus min. drei Elementen, die gegeneinander/miteinander verschiebbar angeordnet sind. Der Stirnstützenschaft (8) ist mit dem Gaszuführenden PI im Bereich des Maskenkörpers (1) verbunden und das Verstellelement (13) mit dem Stirnstützenträger (6) über den Arm (7). Dazwischen ist ein weiteres drittes Verstellelement (14) angeordnet Das Verstellelement (14) kann über zwei Gleitflächen (15, 16) bewegt werden. Durch das zusätzliche Verstellelement (14) ergibt sich ein größerer Verstellbereich. In Fig.6 sind die zwei Gleitflächen (15, 16) parallel angeordnet. Hiermit lässt sich ein größerer Verstellbereich in der Höhe, also in Richtung der X-Achse, erzielen. Fig.7 hingegen zeigt eine gegengleiche Anordnung der Gleitflächen (15, 16). Hiermit lässt sich ein größerer Verstellbereich in der Tiefe, also in Richtung der Y-Achse erzielen. Eine weitere Möglichkeit der Anordnung der Gleitflächen (15, 16) ist in Fig.8 dargestellt. Dieser wäre z.B. für Gesichtskonturen mit einer niedrigen Stirnhöhe aber mit sehr nach vorn stehender Stirn vorteilhaft.

Die Ausführungsformen der Figuren 5 bis 8 können auch glatte Gleitflächen aufweisen, durch welche eine graduelle, nicht gerastete Verstellung ermöglicht wird. Die Fixierung in einer Position erfolgt dann über eine Klemmung.

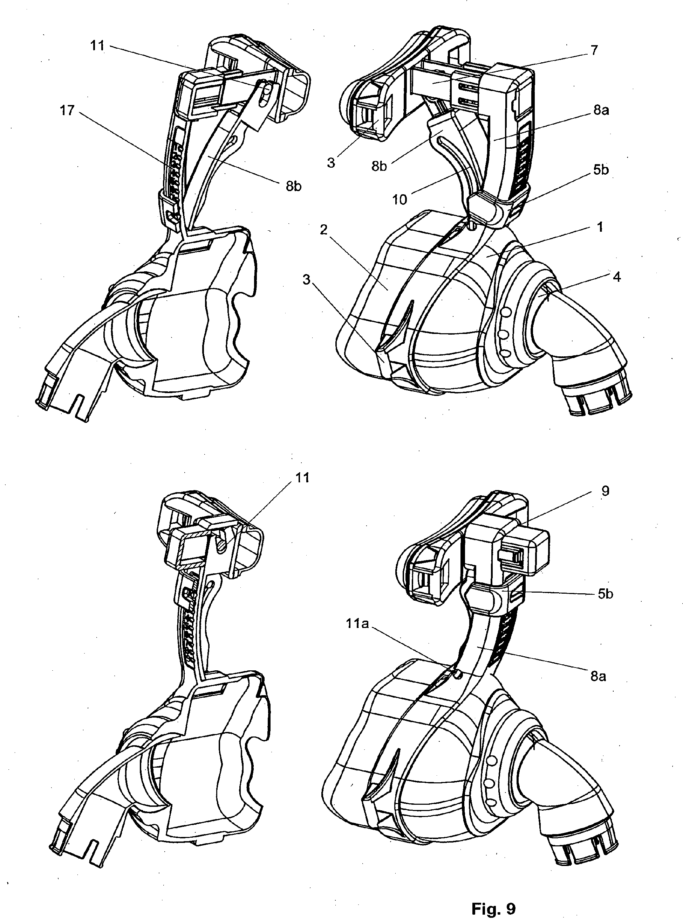

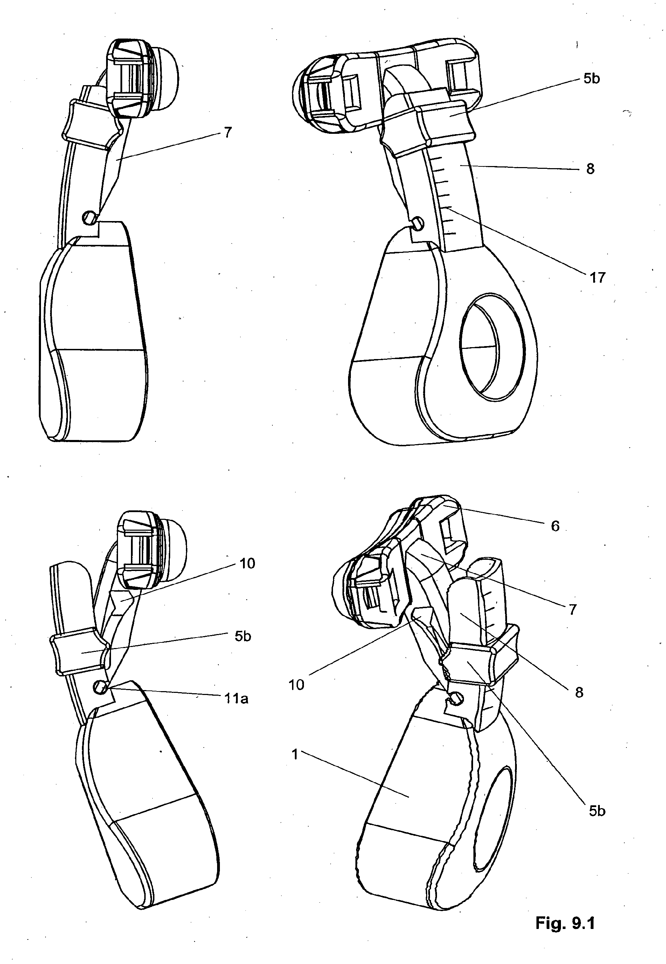

Eine weitere Ausführungsalternative für die Stirnstützenverstellung wird in der Fig.9 dargestellt. Bei dieser Stirnstützenverstellung besteht der Stirnstützenschaft (8) aus zwei Teilen, einem feststehenden (8a), mit dem Maskenkörper (1) verbundenen und einem beweglichen Teil (8b). Das feststehende Teil (8a) des Stirnstützenschaftes weist eine Öffnung (9) auf, in der der Arm des Stirnstützenträgers (7) aufgenommen wird. Der Arm des Stirnstützenträgers (7) besteht aus teleskopartig in- und auseinander bewegbaren Teilen. Der bewegliche Teil des Stirnstützenschaftes (8b) wird am oberen Ende über eine Schnappverbindung (11) an einer Achse im Arm des Stirnstützenträgers mit dem teleskopartigen Arm des Stirnstützenträgers (7) verbunden. Das untere Ende des beweglichen Stirnstützenschaftes (8b) wird über zwei Halteelement (Pins) (11a) mit dem feststehenden Teil (8a) drehbar verbunden und ist so ausgeführt, dass er sich in das feststehende Teil (8a) einfügen kann. Des Weiteren weist der bewegliche Teil (8b) des Stirnstützenschaftes Führungsnuten (10) auf, in denen Führungselemente (Pins) eines Schiebers (5b) greifen. Der Schieber (5b) dient als Verstellelement und bewegt sich auf der Kontur des feststehenden Teiles (8a) des Stirnstützenschaftes in einer gestreckten Bewegung. Durch Bewegen des Verstellelementes (Schiebers) (5b) wird der bewegliche Teil (8b) des Stirnstützenschaftes im feststehenden Teil (8a) bewegt und nimmt den teleskopartigen Arm des Stirnstützenträgers (7) dabei mit. Die Stirnstütze (6) wird dabei horizontal linear vor- und zurückgestellt.

Bevorzugt ist eine mechanische Betätigung des Teleskops, möglich ist aber auch eine hydraulische, pneumatische, etc. Betätigung.

Eine etwas einfachere Ausführungsalternative dieser Stirnstützenverstellung wird in Fig.9.1 dargestellt. Hierbei handelt es sich um eine zweigeteilte Stirnstützenverstellung, die einen Stirnstützenschaft (8) aufweist, der fest mit dem Maskenkörper (1) verbundenen ist und einem beweglichen Stirnstützenträgerarm (7), der sich beim Verstellen der Stirnstütze (6) in dem Stirnstützenschaft (8) bewegt. Die Verbindung zwischen Stirnstützenschaft (8) und Stirnstützenträgerarm (7) erfolgt über zwei Halteelemente (Pins) (11a), die am Stirnstützenträgerarm (7) angeordnet sind und drehbar im Stirnstützenschaft (8) gelagert sind. Der Stirnstützenträgerarm (7) ist schmaler als der Schaft (8) und bewegt sich in diesem vor und zurück.

Das Verstellen erfolgt über das Bewegen in einer gestreckten Bewegung eines Schiebers (5b), der über dem feststehenden Stirnstützenschaft (8) greift und Führungselemente (Pins) aufweist, die in Führungsnuten (10) im Stirnstützenträgerarm (7) greifen und diesen beim Verstellen bewegen.

Die Führungsnuten (10) im Stirnstützenträgerarm (7) laufen nach oben hin leicht schräg aus und bewirken bei leichtem Druckaufwand auf den Schieber (5b) ein Aufspreizen des Schiebers (5b) wodurch dieser sich leicht demontieren lässt. Danach lassen sich die Halteelemente (11a) des beweglichen Stirnstützenträgersarms (7) aus seiner unteren Halterung demontieren.

Zur Montage werden die Halteelemente (11a) des Stirnstützenträgerarms (7) in die Gegenlager im Stirnstützenschaft (8) eingeklickt, der Schieber (5b) kann danach leicht von oben in die Führungsnuten (10) eingeführt werden oder aber die Führungselemente (Pins) des Schiebers (5b) lassen sich durch die Schwenkbewegung des Stirnstützenträgerarms (7) aufweiten und schnappen so wieder in die Führungsnuten (10) ein.

Es ist auch an eine Kennzeichnung (17) der eingestellten Position des Abstützelementes (6) bzw. des Stellgliedes (5b) gedacht. So kann z.B. eine spezielle Einstell-Position, ein Einstell-Bereich oder eine Mittelstellung des Stellbereiches gekennzeichnet werden oder auch jede Raststellung. Dieses kann über eine optische und/oder akustische und/oder taktile Kennzeichnung (17) der eingestellten Position des Abstützelements bzw. des Stellglieds erfolgen. Es können auch mehrere Positionen oder Positionsbereiche im Stellbereich der Vorrichtung zum Abstützen, optisch und/oder akustisch und/oder taktil hervorgehoben sein.

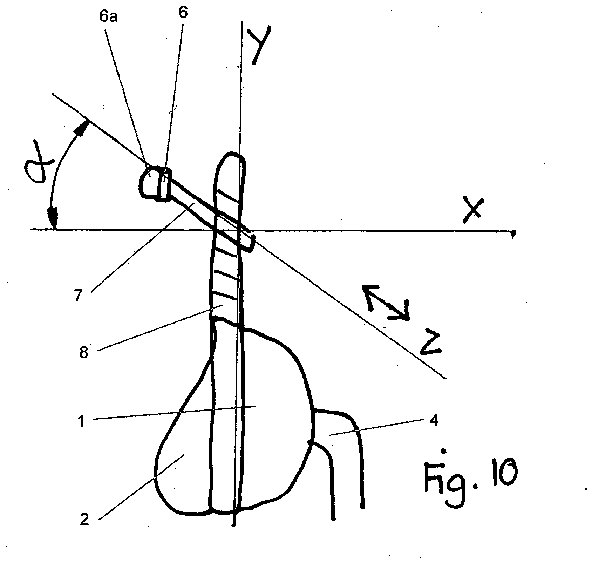

Fig. 10 zeigt die Verstellung der Stirnstütze (6), bei der der Stirnstützenschaft (8) senkrecht zur X-Achse verläuft, hier als Y-Achse dargestellt, und der Stirnstützenträger (6) mit der Stirnauflage (6a) in einem fester Winkel alpha zur X-Achse in Richtung der dargestellten Z-Achse steht. Der Winkel alpha kann zwischen 1° und 89° zur X-Achse betragen. Entlang der Z-Achse ist der Stirnstützenträger (6) variabel verschieb- und arretierbar. Der Winkel zwischen dem Stirnstützenschaft (8) und dem Stirnstützenträger (6) mit der Stirnauflage (6a) bleibt dabei konstant. Beim Verschieben des Stirnstützenträgers (6) entlang der Z-Achse relativ zum Stirnstützenschaft (8) verändert sich der Abstand der Stirnauflage (6a) zur X-Achse derart, dass diese parallel zur Y-Achse gleitend vor- und zurück bewegt wird. Ein vorgegebenes Raster im Stirnstützenschaft (8) ermöglicht eine Änderung der Höhe des Stirnstützenträgers (6) entlang der Y-Achse.

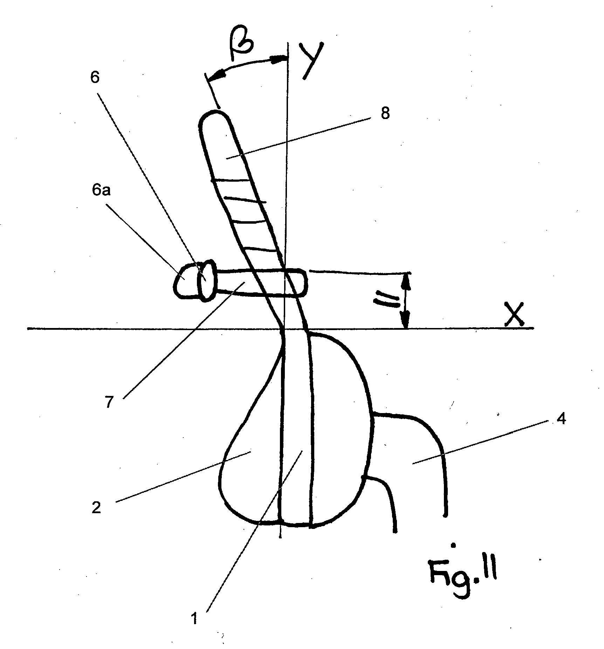

Der Stirnstützenschaft (8) kann nicht nur senkrecht zur X-Achse sein, sondern wie in Fig.11 dargestellt eine leichte Bogenform zum Patientengesicht aufweisen. Der Stirnstützenträger (6) bewegt sich bei der Verstellung linear entlang des Bogens. Durch das ebenfalls im Stirnstützenschaft (8) vorgegebene Höhenraster entsteht ein noch größerer Verstellbereich. Möglich ist es auch den Stirnstützenschaft (8) unter einem festen Winkel beta zur Y-Achse auszuführen. Hierbei ist der Stirnstützenträger (6) parallel zur X-Achse angeordnet. Der Winkel zwischen dem Stirnstützenschaft (8) und dem Stirnstützenträger (6) mit der Stirnauflage (6a) bleibt bei der Verstellung konstant.

Das Verstellen des Stirnpolsters (6a) kann wie oben angeführt über Verschieben und Arretieren des Stirnstützenträgers (6) im Stirnstützenschaft (8) erfolgen oder über Gewindeteile, die zum Verstellen dienen. Hierbei verfügen alle Stirnstützenteile, der Schaft (8), der Arm des Stirnstützenträgers (7) und die Stirnstütze (6) über Gewindeteile. Das Drehen der Gewindeteile ermöglicht die Wahl einer für den Patienten geeignete Position der Stirnauflage (6a) und deren Arretierung.

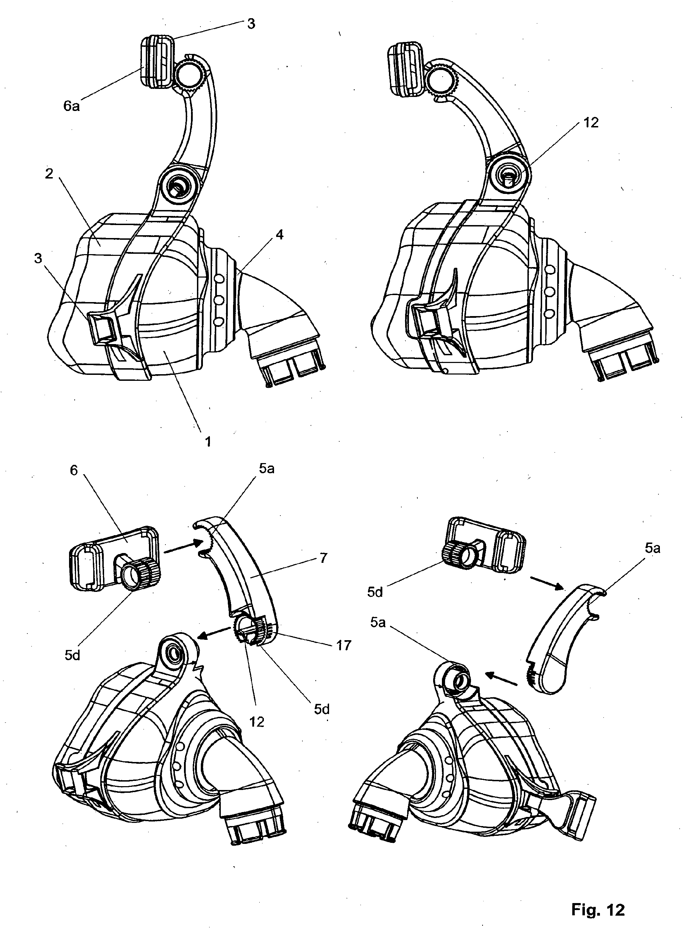

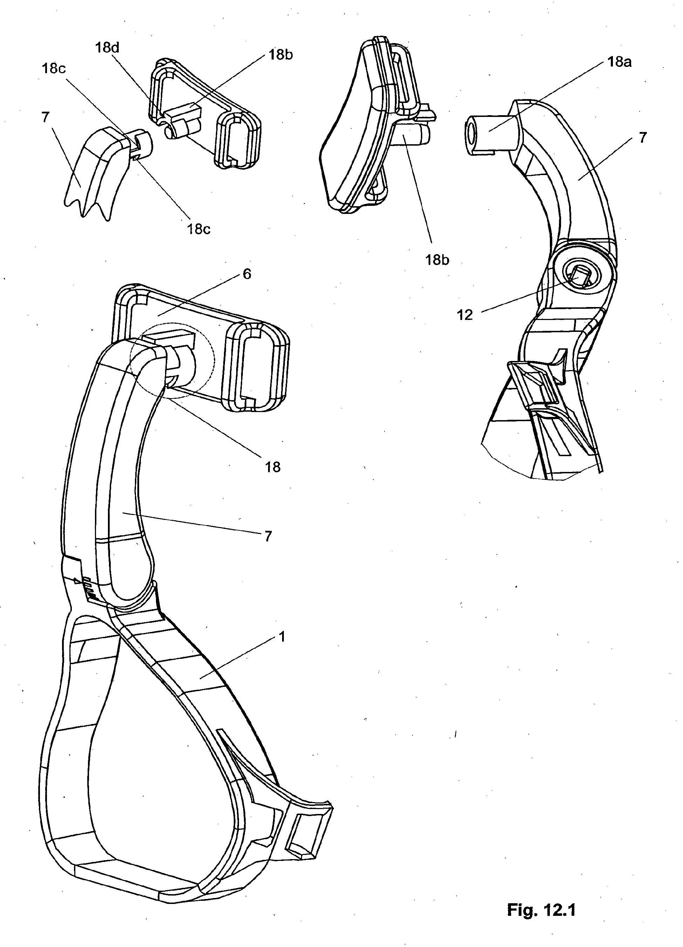

Fig.12: Eine weitere alternative Einstellung der Stirnstütze (6) wird durch eine Ausführungsvariante ermöglicht, deren Verstellung nur im horizontal entkoppelten Zustand möglich ist. Am Maskenkörper (1) befindet sich eine kreisrunde, ringförmig ausgeführte, innen verzahnte Aufnahme(5a), ähnlich einem Zahnkranz, die ein außen verzahntes Gegenstück (5d), welches am unteren Ende des Stirnstützenarms (7) angeordnet ist, aufnehmen kann und so eine zusammengehörige Einheit bilden.

Zur Änderung der Position der Stirnstütze (6) wird diese Verbindung durch leichten Druck auf einen Schnapphaken (12) am Stirnstützenarm (7) gelöst. Die beiden Elemente, Maskenkörper (1) und Stirnstützenarm (7) werden danach in einer relativ zueinander veränderten Lage wieder montiert. Am oberen Ende des Stirnstützenarms (7) befindet sich ebenfalls eine innen verzahnte Aufnahme (5a), die eine Öffnung in Richtung der Stirnstütze (6) aufweist und ein außen verzahntes Gegenstück (5d), welches sich an der Stirnstütze (6) befindet, aufnimmt. Dieses dient zum Anpassen der Stirnstütze (6) mit dem Stirnpolster (6a) an die relativ veränderte Position des Stirnstützenarmes (7). Hierzu wird die Stirnstütze (6) horizontal aus der Aufnahme (5a) gezogen und in einer veränderten Lage wieder montiert. Das außen verzahnte Element (5d) an der Stirnstütze (6) ist vertikal außerhalb der Mitte angeordnet. und ermöglicht so durch ein Verdrehen um 180° eine Höhenveränderung.

Eine alternative Höhenanpassung der Stirnstütze (6) wird in Fig.12.1 dargestellt. Die Verbindung von Stirnstützenarm (7) und Stirnstütze (6) ist ähnlich eines Bajonettverschlusses (18). Am Stirnstützenarm (7) ist eine zylindrische Aufnahme (18a) angebracht. Die zylindrische Aufnahme (18a) weist einen seitlichen hinterschnittenen Anschlag (18c) auf. Die Stirnstütze (6) weist ein zylindrisches Gegenstück (18b) auf, welches in die zylindrische Aufnahme (18a) des Stirnstützenarms (7) passt und einen halbkreisförmigen Anschlagbereich (18d), der in senkrechter Position der Stirnstütze (6) über die zylindrische Aufnahme (18a) geführt wird und durch Drehen um 90° hinter den Anschlag (18c) des Stirnstützenarms (7) fasst und so gehalten wird. Ein seitlicher Anschlagpunkt verhindert das Drehen um 360° der Stirnstütze (6). Durch ein Verdrehen um 180° wird eine Höhenveränderung ermöglicht. Auch in dieser Position wird die Stirnstütze (6) durch den hinterschnittenen Anschlag (18c) am Stirnstützenarm (7) gehalten.

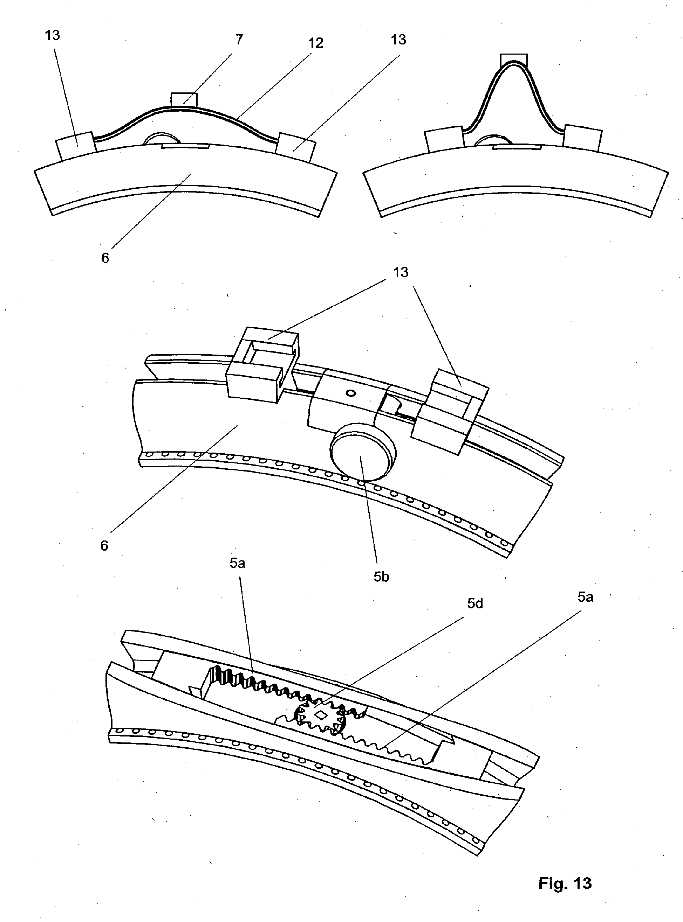

Eine weitere Verstellung der Stirnstütze zum Patientenkopf ist durch ein deformierbares Element (12) ähnlich einer Blattfeder realisiert. Über die Bogenspannung des deformierbaren Elementes (12), welches, wie in Fig.13 dargestellt, im Stirnstützenträger (6) angeordnet ist und durch zwei seitliche Verstellelemente (13) gehalten wird, kann der Abstand verändert werden. Über Rastschlitze, ähnlich einer Verzahnung (5a) in die Rastnasen (Zähne) des Verstellelementes (5d) greifen, wird das deformierbare Elemente (12) bewegt und verändert so den Abstand des Stirnstützenarms (7) zum Patientengesicht. Durch einen Schlitz im Stirnstützenarm (7) wird das deformierbare Element (12) geführt und bildet so eine Verbindung zwischen Stirnstützenarm (7) und Stirnstützenträger (6).

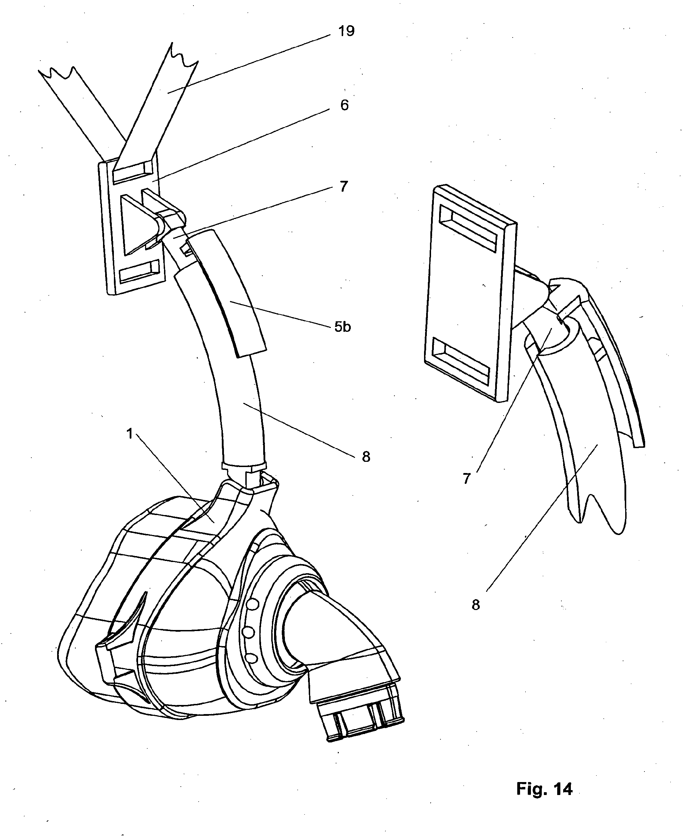

Alternativ zu den Ausführungen mit einer Stirnauflage (6) kann die sichere Befestigung des Patienten Interfaces am Kopf des Patienten auch über eine Abstützung auf dem Kopf des Patienten erfolgen. Die Oberkopfauflage (Fig.14) wird mit der Bänderung (19) und über eine zum Patienten hin gebogenen Verstelleinheit (5b, 7, 8) mit dem verlängerten Schaft des Maskenkörpers (1) verbunden. So kann der Patient das Patienten Interface individuell auf seine Kopfform einstellen. Die Verbindung der Oberkopfauflage (6) mit der Bänderung (19) erfolgt über Klettverbindungen, die leicht zu lösen und zu verstellen sind. Die Verstelleinheit (5b, 7, 8) bietet gerasterte Verstellpunkte für die optimale Einstellung. So bleibt das Gesicht völlig frei und es wird vermieden, dass der Patient durch Auflagepunkte im Gesicht beeinträchtigt wird.

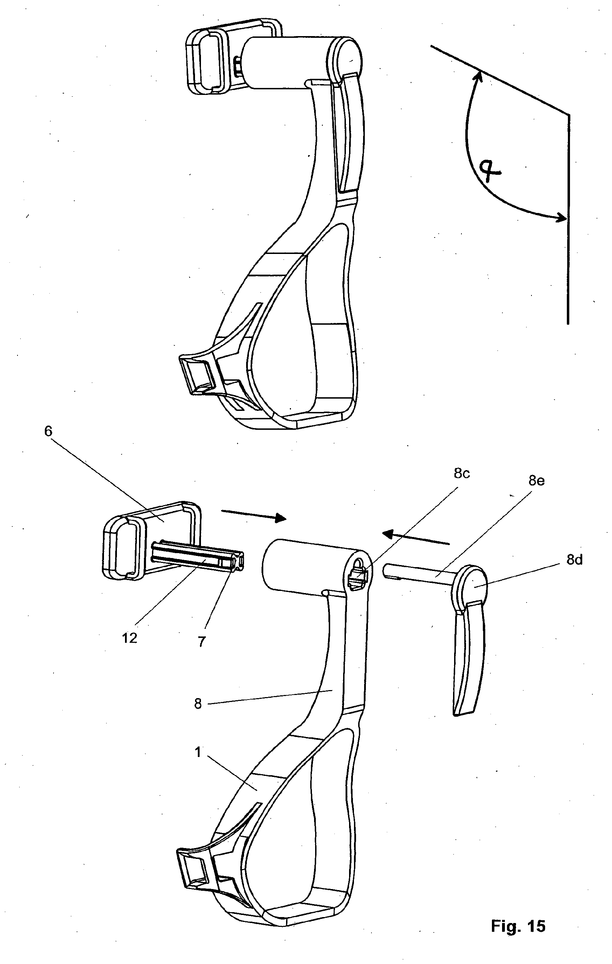

Fig.15: Eine lineare horizontale, stufenlose Einstellung der Stirnstütze (6) wird durch eine Verbindung ermöglicht, bei der der Arm des Stirnstützenträgers (7) in einer Aufnahme (8c) des Stirnstützenschaftes (8) horizontal geführt wird.

Der Stirnstützenarm (7) weist eine annähernd X-förmige Kontur auf und hat zusätzlich seitlich in der X-förmigen Kontur federnde Elemente (12) eingefügt. Die federnden Elemente (12) haben an den Enden einen Haken. Der Stirnstützenschaft (8) ist mit dem Maskenkörper (1) verbunden und weist am oberen Ende einen Zylinder (8c) zur Aufnahme für den Stirnstützenarm (7) auf. Diese Aufnahme (8c) ist bevorzugt horizontal, alternativ auch unter einem Winkel alpha, der zwischen 80° bis 110° liegen kann, zum Stirnstützenschaft (8) angeordnet und nimmt patientenseitig den Arm des Stirnstützenträgers (7) und von der Gegenseite einen Spannhebel (8d) auf. Die Aufnahme (8c) weist innen liegende Führungsnuten, komplementär zum Stirnstützenarm (7) und der Achse des Spannhebels (8e) auf und hat patientenseitig einen Anschlag, hinter den die federnden Elemente (12) des Stirnstützenarms (7) greifen und ein Herausrutschen des Stirnstützenarms (7) verhindern. Die zylindrische Achse des Spannhebels (8e) weist am Ende eine Verdickung auf. Die Verdickung dient zum Klemmen des Stirnstützenarmes (7) in der eingestellten Position. Dieses wird durch Drehen des Hebels (8d) in die Festposition erreicht. In der Festposition bildet der Spannhebel (8d) eine einheitliche Silhouette mit dem Stirnstützenschaft (8). Das Lösen erfolgt durch eine Drehung des Spannhebels (8d) um 90°nach rechts oder links. In dieser Position des Spannhebels (8d) ist die Lage des Stirnstützenarmes (7) stufenlos variierbar.

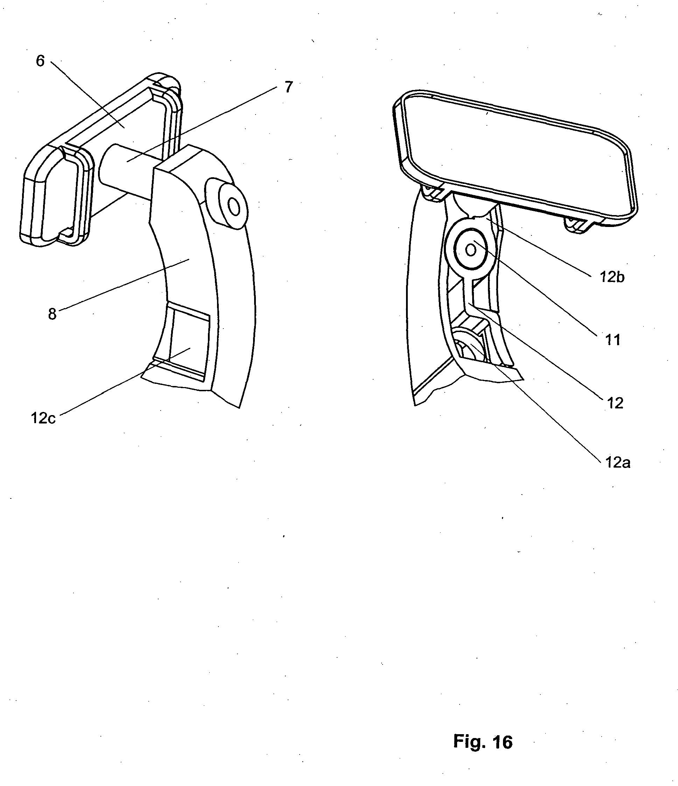

Eine alternative Variante bei der der Arm (7) des Stirnstützenträgers (6) im Stirnstützenschaft (8) stufenlos linear horizontal verschoben wird, ist in Fig.16 dargestellt. Der Stirnstützenschaft (8) ist patientenseitig offen ausgeführt und weist innen liegend eine Spannvorrichtung auf, die den Arm (7) des Stirnstützenträgers (6) im Stirnstützenschaft (8) in einer eingestellten Position klemmt. Das Klemmen des Stirnstützenarmes (7) erfolgt über einen Spannhebel (12), der drehbar im Stirnstützenschaft (8) gelagert (11) ist und durch die Vorspannung eines federnden Elementes (12) in Form eines Ringes (12a), der von innen gegen das untere Ende des Spannhebels (12) drückt. Der Spannhebel (12) drückt dabei mit einer am oberen Ende angesetzten Nase (12b) gegen den Arm (7) des Stirnstützenträgers (6) und hält somit den Arm (7) des Stirnstützenträgers (6) in der gewünschten Position. Zum Verschieben des Armes (7) reicht ein leichter Druck seitlich auf einen Drücker (12c) am Spannhebel (12), der die Vorspannung des federnden Elementes (12a) aufhebt. Der Drücker des Spannhebels (12c) ist durch eine seitliche Öffnung des Stirnstützenschaftes (8) erreichbar. Eine Neigung des Stirnstützenträgers (6) zum Patientengesicht ist nicht erforderlich, da ein aufgesetztes Stirnpolster (6a) (nicht dargestellt) durch seine weiche anpassungsfähige Eigenschaft in jeder Position einen angenehmen Tragekomfort ermöglicht.

Dieses alles sind Ausführungsbeispiele, die einen sicheren, angenehm tragbaren Halt der Vorrichtung zur Beatmung ermöglichen und in ihrer Handhabung einfach für den Patienten zu benutzen sind und dem Patienten helfen, die Vorrichtung zur Beatmung komfortabel über einen längeren Zeitraum zu tragen und das Risiko einer Ablosen der Vorrichtung verhindern.

## Patentansprüche

1. Vorrichtung zur Beatmung, zumindest bestehend aus einem Körper (1), welcher einen Innenraum von einem Außenbereich abgrenzt, einem Bereich zur Abdichtung (2) der Vorrichtung gegen Gesichtspartien eines Patienten, Befestigungselementen (3) zur Positionierung der Vorrichtung am Kopf des Patienten und einem Anschluss zur Zufuhr von Atemgas (4), wobei die Vorrichtung zur Beatmung über ein Stellglied (5) verfügt, das beim Verstellen einen Abstand mindestens eines Teiles der Vorrichtung zu mindestens einem Körperbereich des Patienten verändert, wobei die Vorrichtung eine Stirnstütze (6), einen Stirnstützenträger (7) und einen Stirnstützenschaft (8) aufweist, wobei der Stirnstützenschaft am Maskenkörper integriert ist, **dadurch gekennzeichnet, dass**
der Stirnstützenträger (7) einen Arm aufweist der geschlitzt oder geteilt ist und ein federndes Element (12) mit einem Verstellknopf (5b) aufweist, wobei der Arm seitliche, gebogene Abrollflächen (5a) aufweist, die entweder glatt oder gezahnt sind, wobei das Stellglied (5) das federnde Element (12) mit dem Verstellknopf (5b) und den seitlichen Abrollflächen (5a) umfasst, wobei eine Verstellmöglichkeit der Stirnstütze (6) über das Abrollen der seitliche, gebogenen Abrollflächen (5a) am geschlitzten oder geteilten Arm des Stirnstützenträgers (7) auf jeweils einer entsprechenden glatten oder gezahnten Fläche (5d) außen am Stirnstützenschaft (8) ermöglicht ist und die Fixierung oder Blockierung der Abrollbewegung über eine Geometrie erfolgt, bei der das federnde Element (12) Rastnasen aufweist, die die Abrollbewegung blockieren, indem sie in verschiedene Nuten am Stirnstützenschaft (8) einrasten und so die Stirnstütze (6) in der gewünschten Position fixieren.

## Claims

1. A device for ventilation consisting of at least one body (1) that separates an interior space from an exterior region, a region for sealing (2) the device against parts of a patient's face, fastening elements (3) for positioning the device on the patient's head, and a connection for the supply of respiratory gas (4), wherein the device for ventilation has an adjusting element (5) that, when adjusted, changes a distance of at least part of the device to at least one region of the patient's body, wherein the device has a forehead support (6), a forehead support carrier (7), and a forehead support shaft (8), wherein the forehead support shaft is integrated with the mask body, **characterized in that**
the forehead support carrier (7) has an arm that is slotted or divided and a resilient element (12) with an adjusting knob (5b), wherein the arm has lateral, curved rolling surfaces (5a) that are either smooth or toothed, wherein the adjusting element (5) comprises the resilient element (12) with the adjusting knob (5b) and the lateral rolling surfaces (5a), wherein an adjustability of the forehead support (6) is enabled by the lateral, curved rolling surfaces (5a) on the slotted or divided arm of the forehead support carrier (7) rolling on a corresponding smooth or toothed surface (5d) on the outside of the forehead support shaft (8), and the rolling movement is fixed or blocked by a geometry in which the resilient element (12) has detent lugs that block the rolling movement **in that** they snap into various grooves in the forehead support shaft (8) and thus fix the forehead support (6) in the desired position:

## Revendications

1. Dispositif de ventilation constitué au moins par une coque (1), laquelle sépare une zone intérieure d'une zone extérieure, par une zone assurant l'étanchéité (2) du dispositif par rapport aux parties du visage d'un patient, par des éléments de fixation (3) servant à positionner le dispositif sur la tête du patient et par un raccord d'alimentation en gaz respiratoire (4), dans lequel le dispositif de ventilation dispose d'un actionneur (5), dont le réglage fait varier une distance d'au moins une pièce du dispositif par rapport à au moins une zone corporelle du patient, dans lequel le dispositif présente un appui frontal (6), un support d'appui frontal (7) et un axe d'appui frontal (8), dans lequel l'axe d'appui frontal est intégré à la coque de masque, **caractérisé en ce que**
le support d'appui frontal (7) présente un bras, qui est fendu ou segmenté, et un élément à ressort (12) doté d'un bouton de réglage (5b), dans lequel le bras présente des surfaces de roulement incurvées latérales (5a), qui sont soit lisses soit crénelées, dans lequel l'actionneur (5) comprend l'élément à ressort (12) doté du bouton de réglage (5b) et des surfaces de roulement latérales (5a), dans lequel il est possible de régler l'appui frontal (6) à l'extérieur sur l'axe d'appui frontal (8) en déroulant les surfaces de roulement incurvées latérales (5a) au niveau du bras fendu ou segmenté du support d'appui frontal (7) sur respectivement une surface lisse ou crénelée correspondante (5d) et la fixation ou le blocage du mouvement de déroulement sont réalisés via une configuration géométrique, où l'élément à ressort (12) présente des ergots d'encliquetage, qui bloquent le mouvement de déroulement en s'enclenchant dans diverses rainures sur l'axe d'appui frontal (8) et en fixant ainsi l'appui frontal (6) dans la position souhaitée.
